# EUROPÄISCHE PATENTANMELDUNG

(11) **EP 2 052 759 A1**
(43) Veröffentlichungstag der Anmeldung: **29.04.2009**
(21) Anmeldenummer: 07021063.8
(22) Anmeldetag: 27.10.2007
(51) Int. Cl.: A61N 5/10, A61B 6/03

(54) **Strahlentherapieeinrichtung**

(71) Anmelder: Brenneisen, Werner, 30161 Hannover (DE)
(72) Erfinder: Brenneisen, Werner, 30161 Hannover (DE)
(74) Vertreter: Jabbusch, Matthias

(57) **Zusammenfassung**

Bei ein Vorrichtung zur medizinischen Behandlung von Personen durch ionisierende Strahlung, insbesondere Partikeltherapieanlage, mit zumindest einer Gantry (2), welche mindestens einen Strahlenauslaß für einen Therapiestrahl (3) aufweist, und mit wenigstens einer Bildgebungseinrichtung (12) zur Lagebestimmung der im Behandlungsbereich des Therapiestrahles über einen verstellbaren Patiententisch (6) zu positionierenden Person, ist die Bildgebungseinrichtung (12) mittels eines separaten Rahmengestelles (13) aufgenommen, an dem die Bildgebungseinrichtung (12) sich auf zumindest einer Kreisbahn bewegend gehalten ist. Die sich auf einer Kreisbahn bewegende Bildgebungseinrichtung (12) bildet eine Rotationsachse (14), welche koaxial zu einer durch den Behandlungsbereich verlaufenden isozentrischen Längsachse (15) ausgerichtet ist.

## Beschreibung

Die Erfindung bezieht sich auf eine Vorrichtung zur medizinischen Behandlung von Personen durch ionisierende Strahlung, insbesondere eine Partikeltherapieanlage, mit zumindest einer Gantry, welche mindestens einen Strahlenauslaß für einen Therapiestrahl aufweist, und mit wenigstens einer Bildgebungseinrichtung zur Lagebestimmung der im Behandlungsbereich des Therapiestrahles über einen verstellbaren Patiententisch zu positionierenden Person.

Eine solche Strahlentherapieanlage ist insbesondere aus der EP 0 382 560 A1 bekannt, mit Hilfe dieser eine Person zu Therapiezwecken beispielsweise einer hochenergetischen Photonenstrahlung ausgesetzt wird. Der erzeugte Energiestrahl wird dabei gleichzeitig zur Bildgebung verwendet, in dem zwischen dem Energiestrahlerzeuger und der zu behandelnden Person ein die Strahlungsenergie dämpfendes Blendenteil angeordnet ist. Das Blendenteil weist in einem vorbestimmten Bereich einen Durchbruch auf, durch den ein ungedämpfter Anteil des Energiestrahls auf eine zu behandelnde Körperpartie der Person trifft. Dieser Anteil wird dann durch einen auf der dem Energiestrahlerzeuger abgewandten Körperseite der Person zugeordneten Strahlungsteiler abgeschwächt. Dem Energiestrahlerzeuger ist ein Röntgendetektor gegenüber gestellt, welcher den abgeschwächten Energiestrahl zur Lokalisierung des zu bestrahlenden Körperbereichs für Bildgebungszwecke aufnimmt.

Bei derartigen Strahlentherapieanlagen sind dementsprechend neben der eigentlichen zu behandelnden Körperpartie der Person während der gesamten Strahlenbehandlung stetig die benachbarten, gesunden Körperregionen der Energiestrahlung, wenn auch nur in abgeschwächter Form, ausgesetzt. Mitunter kann bereits die abgeschwächte Strahlung bei sensibel reagierenden Personen in diesen eigentlich gesunden Körperregionen Beeinträchtigungen hervorrufen.

Der Erfindung liegt die Aufgabe zugrunde eine Vorrichtung der vorbezeichneten Gattung dahingehend zu verbessern, daß eine zu behandelnde Person speziell für Bildgebungszwecke einer nicht mehr als absolut notwendigen Strahlenbelastung ausgesetzt wird.

Die Lösung der Aufgabe erfolgt erfindungsgemäß durch eine Vorrichtung mit den Merkmalen des Patentanspruchs 1. Vorteilhafte Weiterbildungen und Ausgestaltungen sind in den Unteransprüchen angegeben.

Bei einer Vorrichtung zur medizinischen Behandlung von Personen durch ionisierende Strahlung, insbesondere einer Partikeltherapieanlage, mit zumindest einer Gantry, welche mindestens einen Strahlenauslaß für einen Therapiestrahl aufweist, und mit wenigstens einer Bildgebungseinrichtung zur Lagebestimmung der im Behandlungsbereich des Therapiestrahles über einen verstellbaren Patiententisch zu positionierende Person, ist nach der Erfindung vorgesehen, daß die Bildgebungseinrichtung mittels eines separaten Rahmengestelles aufgenommen ist, an dem die Bildgebungseinrichtung sich auf zumindest einer Kreisbahn bewegend gehalten ist.

Mit Hilfe einer derartig erfindungsgemäß ausgebildeten Strahlentherapieanlage, welche eine an einem separaten Rahmengestell beweglich gehaltene Bildgebungseinrichtung aufweist, ist durch die konstruktive Trennung der Bildgebungseinrichtung vom insbesondere beweglich an der Gantry ausgebildeten Strahlenauslaß mit Vorteil eine voneinander unabhängige Bewegung möglich. Mittels der entkoppelten Bildgebungseinrichtung, welche sich mehrmals pro Sekunde um ihre Rotationsachse bewegen kann, erfolgt somit stets eine relativ schnelle und vorteilhaft präzise Lagebestimmung der im Behandlungsbereich des Therapiestrahles zu positionierenden Person. Die entsprechend einzeln an ihrem Rahmengestell aufgenommene Bildgebungseinrichtung, welche aus mindestens einem Röntgenstrahler und wenigstens einem Röntgendetektor bestehen kann, kommt zudem nur nach Bedarf zur Anwendung, so daß eine unnötige Strahlenbelastung der gesunden Körperpartien, im Gegensatz zu einer kontinuierlich vorgenommenen Lagebestimmung, vermieden ist. Die Lagebestimmung der zu behandelnden Person kann beispielsweise vorab im Nahbereich des Behandlungsbereiches vorgenommen werden. Die entsprechend eingemessene Person kann dann insbesondere über den verstellbaren Patiententisch in den Behandlungsbereich des Therapiestrahles verfahren werden, um mit der eigentlichen Bestrahlung zu beginnen. Das die Bildgebungseinrichtung aufnehmende Rahmengestell kann sowohl als stationär feste bzw. beweglich aufgestellte Einheit ausgebildet sein, wodurch sich mit Vorteil verschiedene An- bzw. Einbaumöglichkeiten des Rahmengestells in der Strahlentherapieanlage umsetzen lassen.

Die sich insbesondere auf einer spiralförmigen Kreisbahn bewegende Bildgebungseinrichtung bildet eine Rotationsachse aus, welche koaxial zu einer durch den Behandlungsbereich des Therapiestrahles verlaufenden isozentrischen Längsachse ausgerichtet ist. Die koaxiale Ausrichtung der Rotationsachse zu der isozentrischen Längsachse, um die der Strahlenauslaß gegebenenfalls auch auf einer Kreisbahn bewegt werden kann, hat den Vorteil, daß eine bereits eingemessene und vorausgerichtete Person mit ihrer zu behandelnden Körperpartie nur noch durch eine gerichtete Bewegung entlang der deckungsgleich verlaufenden Achsen in eine entsprechende Position unterhalb des Strahlenauslasses zu bringen ist und unmittelbar daran mit dem Therapiestrahl behandelt werden kann. Eine radiale Verschiebung des Patiententisches zu den Achsen, wobei unter Umständen eine unbeabsichtigte Lageänderung der Person eintreten kann, ist nicht bzw. nur bedingt während der Behandlung vorgesehen, wodurch das exakte Positionieren der zu behandelnden Person weiter verbessert ist. Die Bildgebungseinrichtung kann sich zu Bildgebungszwecken auf axial zueinander beabstandeten, radial ausgerichteten Kreisbahnen um die Rotationsachse bewegen, sowie eine spiralförmige Kreisbewegung um die gebildete Rotationsachse ausführen.

Der Strahlenauslaß und ein Röntgenstrahler, welcher Teil der Bildgebungseinrichtung ist, sind in einer zur isozentrischen Längsachse senkrecht verlaufenden Ebene angeordnet, wobei deren Strahlachsen während der Strahlenbehandlung in einem Winkel zwischen 70° und 110° zueinander fixiert sind. Mit der Anordnung des Strahlenauslasses und einem Röntgenstrahler der Bildgebungseinrichtung in einer Ebene zur isozentrischen Längsachse der erfindungsgemäßen Vorrichtung kann auf vorteilhafte Weise die Lokalisierung bzw. Lagebestimmung der zu behandelnden Person und deren Strahlenbehandlung in unmittelbarerer Abfolge aufeinander vorgenommen werden, ohne daß die Person zwischenzeitlich mit Hilfe des Patiententisches verfahren werden muß. Um eine Ablenkung bzw. eine Beeinflussung des Therapiestrahles während der Behandlung ausschließen zu können, sind die Strahlachsen des Strahlenauslasses und des Röntgenstrahlers in einem Winkel zwischen 70° und 110° zueinander fixiert. Die Bildgebungseinrichtung weist dementsprechend zwischen dem Röntgenstrahler und dem ihm gegenübergestellten Röntgendetektor in diesem bevorzugten Winkelbereich eine Lücke auf, durch die der Therapiestrahl auf die Person einwirken kann. Durch das Fixieren der Strahlachsen zueinander erfolgt bei einer Drehung des Strahlenauslasses eine entsprechend gekoppelte Nachführbewegung der Bildgebungseinrichtung. Es ist ebenfalls denkbar, während der Behandlung und bei einer Drehung des Strahlenauslasses um die Längsachse gegebenenfalls gleichzeitig eine kontrollierende Lagebestimmung der Person durchzuführen.

Es liegt des weiteren im Rahmen der Erfindung, daß das Rahmengestell mit seiner Bildgebungseinrichtung parallel zu deren Rotationsachse schiebebeweglich gehalten ist. Eine Verschiebung des Rahmengestelles mit seiner Bildgebungseinrichtung entlang der Rotationsachse hat insbesondere den Vorteil, daß der Abstand zwischen dem Behandlungsbereich und der während einer Strahlenbehandlung im Abstand zum Behandlungsbereich positionierten Bildgebungseinrichtung verringert werden kann, derart, daß sich das Rahmengestell möglicherweise bis direkt in den Behandlungsbereich unterhalb des vorzugsweise ebenfalls auf einer Kreisbahn beweglichen Strahlenauslasses der Gantry verfahren läßt. Somit kann die Lagebestimmung mit Vorteil unmittelbar im Behandlungsbereich vorgenommen werden. Über die zu diesem Zweck insbesondere unmittelbar am Rahmengestell und dessen Aufstellfläche ausgebildete Schiebeführung ist des weiteren eine konstruktiv vorteilhafte Möglichkeit zur Umsetzung einer achsparallelen Bewegung der Bildgebungseinrichtung gegeben. In diesem Zusammenhang kann es notwendig sein, den Strahlenauslaß an der Gantry beweglich anzuordnen, um diesen gegebenenfalls aus dem Schiebeweg des Rahmengestelles herausnehmen zu können.

Nach einer Weiterbildung der Erfindung ist vorgesehen, daß das Rahmengestell mit Antriebsmitteln für eine zwangsgeführte Bewegung ausgerüstet ist. Der Einsatz von Antriebsmitteln, wie zum Beispiel Elektromotoren, hat den Vorteil, daß das Rahmengestell vorteilhaft einfach und ohne zusätzlichen Kraftaufwand entlang der Führungsflächen der Schiebeführung verschoben werden kann. Des weiteren läßt sich mit Hilfe der Antriebsmittel eine relativ genaue bzw. exakte Positionierung der Bildgebungseinrichtung zu Bildgebungszwecken vornehmen. Als Antriebsmittel können insbesondere winkelgesteuerte Schrittmotoren eingesetzt werden, die mit einer beispielsweise als Getriebe ausgebildeten Übertragungseinrichtung, welche die durch den Motor erzeugte Drehbewegung vorteilhaft umwandelt bzw. übersetzt, gekoppelt sein können.

Am Rahmengestell ist ein dem Strahlenauslaß für den Therapiestrahl zugeordneter Strahlendetektor angeordnet, mit Hilfe dem auf vorteilhafte Weise eine Aussage über die Stärke der über den Strahlenauslaß abgegebenen Strahlung getroffen werden kann. Die insbesondere über den Strahlenauslaß abgegebenen Partikelstrahlung erzeugt Neutronen, die sich weiter in Richtung der austretenden Strahlung ausbreiten und mit Vorteil auf dem den Strahlenauslaß gegenübergestellten Strahlendetektor treffen und zum Zwecke der Dosimetrie gemessen werden können.

Das Rahmengestell mit seiner Bildgebungseinrichtung und der Patiententisch, welcher zur definierten Lagerung der zu behandelnden Person im Behandlungsbereich dient, weisen jeweils mindestens einen Meßgrößen erfassenden Sensor auf. Durch die Verwendung von Sensoren ist insbesondere eine vorteilhafte Informationsgewinnung über bestimmte Meßgrößen am Rahmengestell und am Patiententisch sichergestellt. Mit Hilfe jedes Sensors kann gleichzeitig die Umwandlung der Meßgrößen in geeignete elektrische Ausgangssignale erfolgen, wobei jeder Sensor noch mit Vorteil eine Signalaufbereitung, in Form einer Verstärkung bzw. einer Filterung, aufweisen kann. Die eingesetzten Sensoren können beispielsweise als Wegstreckensensoren ausgebildet sein, mittels denen dementsprechend eine exakte Angabe über die durch das Rahmengestell und den Patiententisch angefahrenen Positionen möglich ist.

Die Sensoren sind vorzugsweise über eine Einrichtung zur Meßdatenverarbeitung miteinander verknüpft, mit Hilfe der stets eine zeitnahe Auswertung der abgefragten Meßsignale möglich ist. Auf Basis der regelmäßig übermittelten Daten kann beispielsweise mit Vorteil ein kontinuierlicher Überwachungsprozeß sowohl bei der Lagebestimmung durch die Bildgebungseinrichtung als auch während der mit dem Therapiestrahl durchzuführenden Behandlung der Person erfolgen. Es kann sich dabei um eine externe Einrichtung zur Datenverarbeitung handeln, wobei zum Beispiel eine handelsverfügbare Rechnereinheit zur Anwendung kommen kann.

Es ist des weiteren vorgesehen, daß die Bildgebungseinrichtung als konventioneller Computertomograph ausgebildet ist, der sich auf vorteilhaft einfache Weise mit einer Gantry einer Partikeltherapieanlage kombinieren läßt. Der insbesondere mittels des üblichen Computertomographen erzeugte, schmale Fächerstrahl, welcher einen Strahlungswinkel von vorzugsweise etwa 120° aufweist, bietet vorteilhafte Möglichkeiten für eine Projektionsradiographie, eine Volumen-Computertomographie sowie die herkömmliche Aufnahme des zu behandelnden Körpers in Form von tomographischen Scheiben. Durch die Verwendung eines für Bildgebungszwecke über die Bildgebungseinrichtung abgegebenen Fächerstrahles läßt sich zudem der Anteil an Streustrahlung verringern. Somit ist trotz einer vorteilhaft niedrigen Strahlungsintensität des erzeugten Energiestrahles eine verbesserte Qualität der darüber zu erzeugenden Aufnahmen gewährleistet. Es ist ebenfalls denkbar, als Bildgebungseinrichtung einen Positronemissionstomographen einzusetzen bzw. eine Bildgebungseinrichtung zu verwenden, welche die Positronenemissionstomographie und die Computertomographie in einem Gerät vereint.

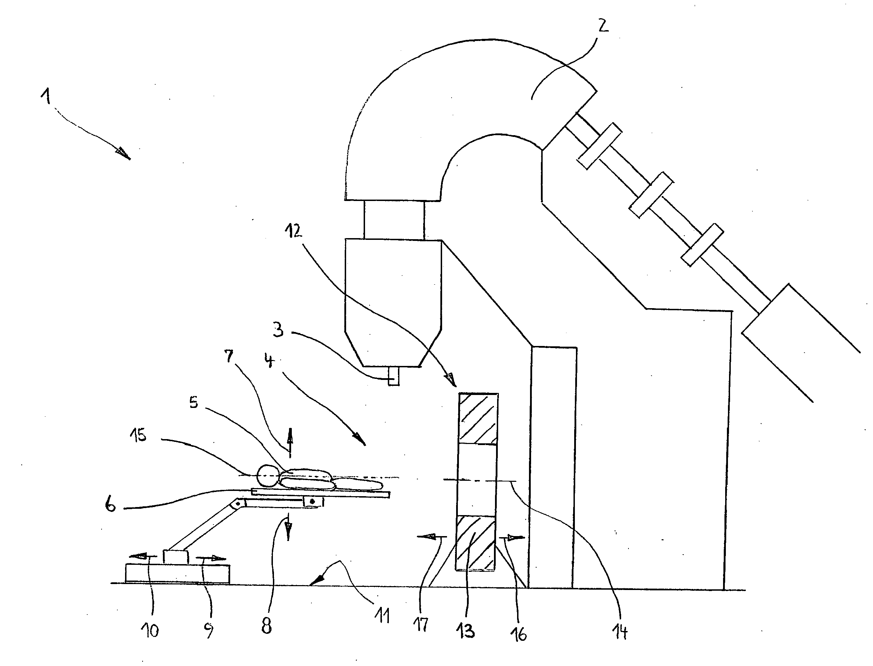

Ein mögliches Ausführungsbeispiel einer Vorrichtung zur Behandlung von Personen, aus dem sich weitere erfinderische Merkmale ergeben, ist in der Zeichnung dargestellt.

Die einzige Figur zeigt eine Ansicht einer erfindungsgemäß ausgebildeten Partikeltherapieanlage.

Mit 1 ist eine Partikeltherapieanlage bezeichnet, die zumindest eine Gantry 2 aufweist, welche als Tragvorrichtung für zum Beispiel einen Strahlenauslaß 3 dient. Über den Strahlenauslaß 3 tritt ein in der Partikeltherapieanlage 1 mittels eines nicht näher beschriebenen Teilchenbeschleunigers erzeugter Therapiestrahl zur Behandlung einer im Behandlungsbereich 4 gelagerten Person 5 aus. Zur Lagerung, insbesondere zur Positionierung der Person 5 im Behandlungsbereich 4 des vorzugsweise als Partikelstrahl vorliegenden Therapiestrahles dient ein Patiententisch 6, der entsprechend den Richtungspfeilen 7, 8, 9, 10 sowohl in der Höhe als auch hinsichtlich seiner Position über der Aufstellfläche 11 verstellbar bzw. verfahrbar ausgebildet ist. Um die für eine Strahlentherapie in jedem Fall notwendige Lagebestimmung der auf dem Patiententisch 6 gelagerten Person durchführen zu können, weist die Partikeltherapieanlage 1 des weiteren eine Bildgebungseinrichtung 12 auf, die mittels einem separaten Rahmengestell 13 aufgenommen ist. Die Bildgebungseinrichtung 12 ist auf zumindest einer Kreisbahn beweglich gehalten, wobei die sich darüber ausbildende Rotationsachse 14 vorzugsweise koaxial zur isozentrischen Längsachse 15 der Partikeltherapieanlage 1 ausgerichtet ist, die durch den Behandlungsbereich 4 verläuft und um diese der Strahlenauslaß 3 beweglich gelagert ist. Dementsprechend wird die zur Lagebestimmung in den freien Querschnitt der Bildgebungseinrichtung 12 eingeschobene Person 5 nach ihrem erfolgten Einmessen bzw. Einscannen mit Hilfe des Patiententisches 6 nur noch entlang der deckungsgleich ausgerichteten Rotationsachse 14 und der Längsachse 15 verschoben, so daß stets eine exakte Positionierung der Person 5 im Behandlungsbereich 4 gewährleistet ist. Es besteht ebenso die Möglichkeit, daß das Rahmengestell 13 mit seiner Bildgebungseinrichtung 12 über daran angeordnete Antriebsmittel parallel zur Rotationsachse der Bildgebungseinrichtung 12, wie die entsprechenden Richtungspfeile 16, 17 angeben, eine zwangsgeführte Bewegung vollzieht und sich darüber der Abstand zum Behandlungsbereich 4 des Therapiestrahles im Bedarfsfall verringern läßt.

## Patentansprüche

1. Vorrichtung zur medizinischen Behandlung von Personen durch ionisierende Strahlung, insbesondere Partikeltherapieanlage, mit zumindest einer Gantry, welche mindestens einen Strahlenauslaß für einen Therapiestrahl aufweist, und mit wenigstens einer Bildgebungseinrichtung zur Lagebestimmung der im Behandlungsbereich des Therapiestrahles über einen verstellbaren Patiententisch zu positionierenden Person,
**dadurch gekennzeichnet,**
**daß** die Bildgebungseinrichtung (12) mittels eines separaten Rahmengestelles (13) aufgenommen ist, an dem die Bildgebungseinrichtung (12) sich auf zumindest einer Kreisbahn bewegend gehalten ist.

2. Vorrichtung nach Anspruch 1, **dadurch gekennzeichnet, daß** mittels der sich auf einer Kreisbahn bewegenden Bildgebungseinrichtung (12) eine Rotationsachse (14) ausgebildet ist, welche koaxial zu einer durch den Behandlungsbereich (4) verlaufenden isozentrischen Längsachse (15) ausgerichtet ist.

3. Vorrichtung nach einem der Ansprüche 1 und 2, **dadurch gekennzeichnet, daß** der Strahlenauslaß (3) und ein Röntgenstrahler, welcher Teil der Bildgebungseinrichtung (12) ist, in einer zur isozentrischen Längsachse (15) senkrecht verlaufenden Ebene angeordnet sind, wobei deren Strahlachsen während der Strahlenbehandlung in einem Winkel zwischen 70° und 110° zueinander fixiert sind.

4. Vorrichtung nach einem der Ansprüche 1 bis 3, **dadurch gekennzeichnet, daß** das Rahmengestell (13) mit seiner Bildgebungseinrichtung (12) parallel zu deren Rotationsachse (14) schiebebeweglich gehalten ist.

5. Vorrichtung nach einem der Ansprüche 1 bis 4, **dadurch gekennzeichnet, daß** das Rahmengestell (13) mit Antriebsmitteln für eine zwangsgeführte Bewegung ausgerüstet ist.

6. Vorrichtung nach einem der Ansprüche 1 bis 5, **dadurch gekennzeichnet, daß** am Rahmengestell (13) ein dem Strahlenauslaß (3) für den Therapiestrahl zugeordneter Strahlendetektor angeordnet ist.

7. Vorrichtung nach einem der Ansprüche 1 bis 6, **dadurch gekennzeichnet, daß** das Rahmengestell (13) mit seiner Bildgebungseinrichtung (12) und der Patiententisch (6) jeweils mindestens einen Meßgrößen erfassenden Sensor aufweisen.

8. Vorrichtung nach Anspruch 7, **dadurch gekennzeichnet, daß** die Sensoren über eine Einrichtung zur Meßdatenverarbeitung miteinander verknüpft sind.

9. Vorrichtung nach einem der Ansprüche 1 bis 8, **dadurch gekennzeichnet, daß** die Bildgebungseinrichtung (12) als Computertomograph ausgebildet ist.
